Europäisches Patentamt

(19) European Patent Office   (11) Veröffentlichungsnummer: **0 023 918**
**B1**
Office européen des brevets

(12) **EUROPÄISCHE PATENTSCHRIFT**

(45) Veröffentlichungstag der Patentschrift:     (51) Int. Cl.³: **A 61 B 3/00,** A 61 B 3/10
16.05.84

(21) Anmeldenummer: 80900400.5

(22) Anmeldetag: **12.01.80**

(86) Internationale Anmeldenummer:
**PCT/DE 80/00003**

(87) Internationale Veröffentlichungsnummer:
**WO 80/01642 (21.08.80** Gazette 80/19)

(54) **KOMBINATIONSGERÄT FÜR AUGENUNTERSUCHUNG.**

(30) Priorität: **16.02.79 DE 2905915**     (73) Patentinhaber: **Firma Carl Zeiss, Postfach 1369/1380, D-7082 Oberkochen (DE)**

(43) Veröffentlichungstag der Anmeldung:
**18.02.81 Patentblatt 81/7**     (72) Erfinder: **SCHULZ, Kurt, Mozartweg 20, D-7082 Oberkochen (DE)**
Erfinder: **MAGLICA, Peter, Lerchenstrasse 1, D-7082 Oberkochen (DE)**

(45) Bekanntmachung des Hinweises auf die Patenterteilung:
**16.05.84 Patentblatt 84/20**

(84) Benannte Vertragsstaaten:
**CH FR GB**

(56) Entgegenhaltungen:
**CH - A - 396 448**
**DE - B - 1 086 457**
**DE - B - 1 125 202**
**FR - A - 2 345 978**
**GB - A - 913 337**
**US - A - 4 102 565**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Kombinationsgerät für Augenuntersuchung

Die Erfindung betrifft ein Kombinationsgerät für Augenuntersuchung, bestehend aus einer binokularen Fernrohrlupe mit Hauptobjektiv und aus einem Untersuchungsgerät opthalmologischer Art, wobei anstelle des Hauptobjektivs das ophthalmologische Gerät einsetzbar ist. Bei ophthalmologischen Geräten geht der Trend der Entwicklung zu Kombinationsgeräten, mit denen ein rationelles Arbeiten bei geringsten Raumbedarf möglich ist.

Aus der DE-C-2 614 273 ist bekannt, ein Ophthalmometer mit einer binokularen Fernrohrlupe derart zu kombinieren, daß anstelle des Hauptobjektivs der binokularen Fernrohrlupe das optische System des Ophthalmometers einsetzbar ist. Ein derartiges Kombinationsgerät hat den Nachteil, daß bei der Umrüstung von Spaltlampe auf Ophthalmometer und umgekehrt das Hauptobjektiv aus einem Verbindungsteil gelöst und durch den Ophthalmometerzusatz ersetzt werden muß. Das Auf- und Abmontieren der Geräteteile wird nur von einem Benutzerkreis toleriert, der den Wechsel nicht zu oft vorzunehmen hat.

Der Erfindung liegt deshalb die Aufgabe zugrunde, ein Kombinationsgerät anzugeben, das einen schnellen Wechsel von einem Diagnosegerät zum andern, beispielsweise von einer Spaltlampe zum Ophthalmometer, ermöglicht.

Die Aufgabe wird erfindungsgemäß dadurch gelöst, daß das Hauptobjektiv der binokularen Fernrohrlupe in einer um eine senkrecht zum Strahlengang verlaufende Horizontalachse schwenkbar gelagerten Fassung montiert ist, daß die Fassung mit einem Verbindungsstück zur gleichzeitigen Aufnahme des ophthalmologischen Gerätes ausgerüstet ist, und daß wahlweise das Hauptobjektiv oder das ophthalmologische Gerät in den Strahlengang einschwenkbar ist.

Befindet sich das Hauptobjektiv in seiner Arbeitsstellung im optischen Strahlengang, so wirkt die binokulare Fernrohrlupe mit einer Spaltleuchte zusammen und bildet ein Spaltlampengerät.

In einem zweckmäßigen Ausführungsbeispiel der Erfindung ist die Spaltleuchte um eine Vertikalachse schwenkbar angeordnet und kann in den Strahlengang der binokularen Fernrohrlupe ein- und ausgeschwenkt werden. Das an der Fassung des Hauptobjektivs befestigte Verbindungsstück ist zweckmäßigerweise als Aufnahmeschwalbe ausgeführt. Ein zu dieser Aufnahmeschwalbe passendes Einsatzstück kann an einem mit dem Kombinationsgerät verbindbaren Ophthalmometerteil befestigt sein.

Ist ein derartiges Ophthalmometerteil mit der Objektivfassung der binokularen Fernrohrlupe so verbunden, daß es beim Hochklappen des Objektivs in dessen Ruhestellung selbst in seine Arbeitsstellung in den Strahlengang geschwenkt wird, dann ist das Gerät als vollwertiges Ophthalmometer zu verwenden. Ein in die Aufnahmeschwalbe der Hauptobjektivfassung passendes Gegenstück kann aber auch an einem anderen opthalmologischen Gerät, beispielsweise einem Endothel-Mikroskop oder an einer Placido-Lichtquelle befestigt sein, so daß — zusammen mit der binokularen Fernrohrlupe — weitere opthalmologische Untersuchungsgeräte gewonnen werden können.

In einem vorteilhaften Ausführungsbeispiel hat die Fassung des Hauptobjektivs einen rechteckigen Umfang, dessen Oberseite einen zweiteiligen, um je eine Horizontalachse schwenkbaren Schutzdeckel trägt, dessen vorderer Teil in einer aus dem optischen Strahlengang herausgeschwenkten Ruhestellung des Hauptobjektivs dieses bedeckt.

Zweckmäßigerweise ist die binokulare Fernrohrlupe an einem Träger befestigt, der mit einem Loch zur Aufnahme eines entsprechend geformten Ansatzstückes des nach unten schwenkbaren ophthalmologischen Gerätes oder Teilgerätes versehen ist. Dadurch ist dieses Gerät in seiner Ruhelage fixiert.

Außerdem ist es vorteilhaft, im Träger Rohre zur Aufnahme von Stromversorgungskabeln vorzusehen. Diese können mit der Ophthalmometerleuchte und einem Mikroschalter für die Spaltleuchte verbunden sein.

Die mit der Erfindung erzielten Vorteile bestehen insbesondere darin, daß das aus dem Strahlengang in seine Ruhelage geschwenkte Geräteteil im Raum vor dem Patientenauge den Ophthalmologen nicht behindert und daß der Wechsel von einem Untersuchungsgerät zum anderen praktisch nur einen Handgriff erfordert. Ein Ausführungsbeispiel der Erfindung ist in der Zeichnung dargestellt und wird im folgenden näher beschrieben. Es zeigt

Fig. 1 eine seitliche Ansicht auf das Gerät mit in den Strahlengang eingeschwenktem Hauptobjektiv der binokularen Fernrohrlupe;

Fig. 2 eine Teilansicht auf die Seite des Gerätes mit in den Strahlengang eingeschwenktem Ophthalmometerzusatz;

Fig. 3 einen Schnitt entlang der Linie III-III der Fig. 1 in vergrößerter Darstellung.

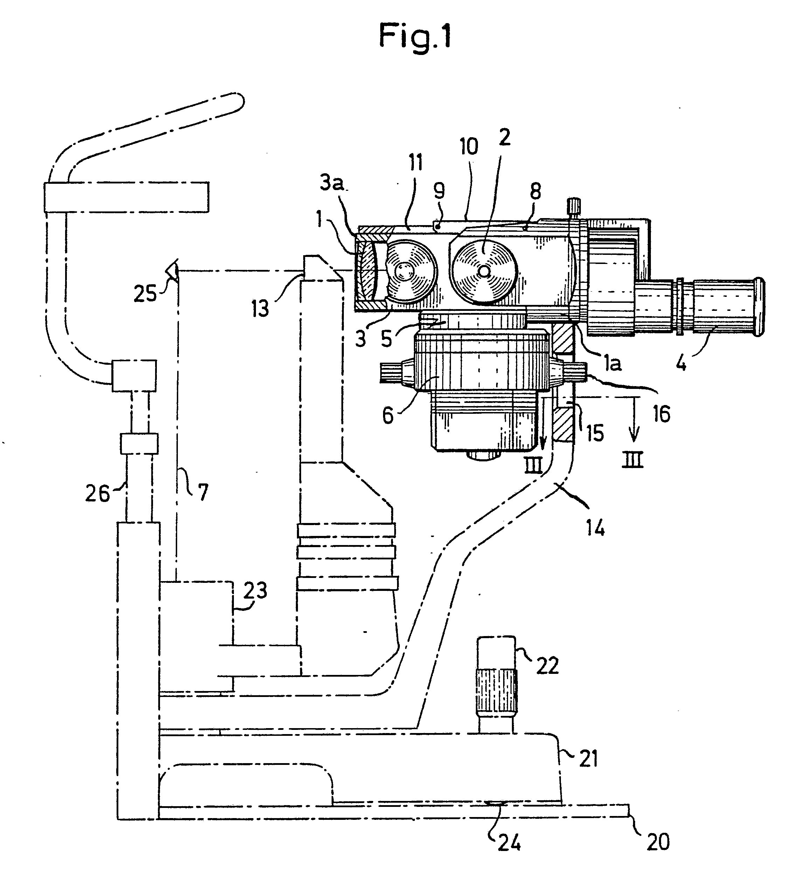

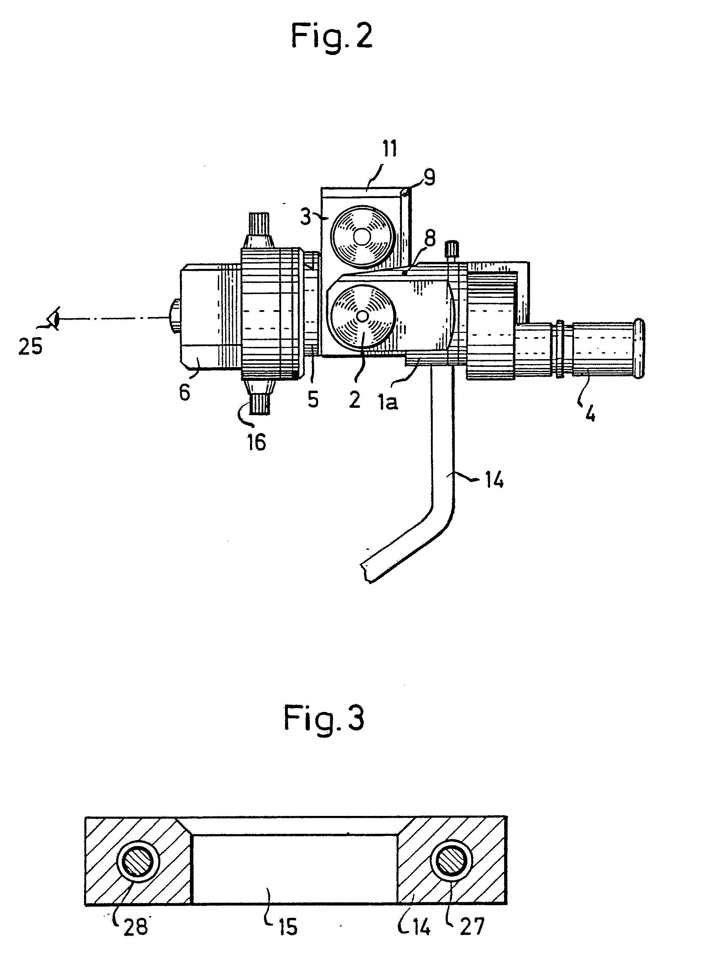

In der Fig. 1 ist mit 20 die Grundplatte des Instrumentes bezeichnet. Auf der Grundplatte befindet sich ein Schlitten 21, der beim Schwenken des Steuerhebels 22 über Kugeln 24 in der Horizontalen verstellt werden kann. Beim Drehen des Steuerknüppels 22 bewegt sich die Instrumentensäule 23 in vertikaler Richtung. Mit der Instrumentensäule verbunden ist der Träger 14 für die binokulare Fernrohrlupe und die Spaltleuchte 13, die um eine mit 7 bezeichnete Vertikalachse schwenkbar ist. Links von der Spaltleuchte befindet sich eine Stütze 26 zur Fixierung des Patientenkopfes mit dem Auge 25. Die einzelnen Teile dieser Kopfstütze sind nicht näher bezeichnet. Die Okularstutzen 4 der binokularen Fernrohrlupe sind über einen feststehenden Teil 1a des Objektivtubus mit dem Träger 14 verbunden, der seinerseits an der Säule 23 befestigt ist. In der in Fig. 1 dargestellten Spaltlampen-Kombination der Erfindung befindet sich das Hauptobjektiv 1 der binokularen Fernrohrlupe im optischen Strahlengang (Arbeitsstellung). Das Hauptobjektiv 1 ist in der Fassung 3, 3a montiert, deren Außenumfang rechteckig ist. Auf der Oberseite der Objektivfassung befindet sich ein zweiteiliger Schutzdeckel, dessen Teile 10, 11 um die horizontalen Achsen, 8, 9 schwenkbar sind. Auf der Unterseite der Objektivfassung ist die Aufnahmeschwalbe 5 befestigt. In der Arbeitsstellung der Spaltlampe des Kombinationsgerätes ist das an der Aufnahmeschwalbe 5 befestigte Ophthalmometerteil 6 nach unten in seine Ruhestellung geklappt und rastet mit dem Ansatzstück 16 in das Loch 15 des Trägers 14 ein, wodurch es in dieser Lage fixiert ist. In der Abbildung der Fig. 2 ist das Hauptobjektiv in seiner Fassung 3, 3a um eine Horizontalachse 2 nach oben in seine Ruhelage geklappt und durch den Schutzdeckel 11 abgedeckt. In den optischen Strahlengang eingeschwenkt ist das Ophthalmometerteil 6 und befindet sich damit in seiner Arbeitsstellung. Anstelle des Ophthalmometerteils 6 können in die Aufnahmeschwalbe 5 andere Geräteteile eingesetzt und in ihrem Gebrauch mit der Spaltlampe durch Ein- und Ausschwenken kombiniert werden, so z. B. ein Endothel-Mikroskop oder eine Placido-Lichtquelle. In der Schnittdarstellung der Fig. 3 sind die in den Träger 14 eingegossenen Rohre zur Führung von Stromversorgungskabeln mit 27 und 28 bezeichnet.

## Patentansprüche

1. Kombinationsgerät für Augenuntersuchung, bestehend aus einer binokularen Fernrohrlupe mit Hauptobjektiv (1) und einem ophthalmologischen Untersuchungsgerät (6), wobei anstelle des Hauptobjektivs (1) das ophthalmologische Gerät (6) einsetzbar ist, dadurch gekennzeichnet, daß das Hauptobjektiv (1) der binokularen Fernrohrlupe in einer um eine senkrecht zum Strahlengang verlaufenden Horizontalachse (2) schwenkbar gelagerten Fassung (3, 3a) montiert ist, daß die Fassung (3, 3a) mit einem Verbindungsstück (5) zur gleichzeitigen Aufnahme des ophthalmologischen Gerätes (6) ausgerüstet ist, und daß wahlweise das Hauptobjektiv (1) oder das ophthalmologische Gerät (6) in den Strahlengang einschwenkbar ist.

2. Kombinationsgerät nach Anspruch 1, dadurch gekennzeichnet, daß bei in den Strahlengang eingeschwenktem Hauptobjektiv (1) (Fig. 1) die binokulare Fernrohrlupe mit einer Spaltlampe (13) zusammenwirkt.

3. Kombinationsgerät nach Anspruch 2, dadurch gekennzeichnet, daß die Spaltlampe (13) um eine Vertikalachse (7) schwenkbar ist.

4. Kombinationsgerät nach Anspruch 1, dadurch gekennzeichnet, daß das Verbindungsstück (5) als Schwalbenschwanzverbindung ausgeführt ist.

5. Kombinationsgerät nach Anspruch 1, dadurch gekennzeichnet, daß am Verbindungsstück (5) ein Ophthalmometerteil (6) befestigt ist.

6. Kombinationsgerät nach Anspruch 1, dadurch gekennzeichnet, daß am Verbindungsstück (5) ein Endothel-Mikroskop befestigt ist.

7. Kombinationsgerät nach Anspruch 1, dadurch gekennzeichnet, daß am Verbindungsstück (5) ein Keratoskop nach Placido befestigt ist.

8. Kombinationsgerät nach Anspruch 1, dadurch gekennzeichnet, daß die Fassung (3, 3a) des Hauptobjektivs (1) rechteckigen Umfang hat und auf der Oberseite (3a) einen zweiteiligen Schutzdeckel (10, 11) trägt, dessen Teile jeweils um eine Horizontalachse (8, 9) schwenkbar sind, wobei der vordere Teil (11) das Hauptobjektiv (1) bedeckt, wenn es aus dem Strahlengang herausgeschwenkt ist (Fig. 2).

9. Kombinationsgerät nach Anspruch 1, dadurch gekennzeichnet, daß die binokulare Fernrohrlupe an einem Träger (14) befestigt ist, der mit einem Loch (15) zur Aufnahme eines entsprechend geformten Ansatzstückes (16) des ophthalmometrischen Gerätes (6) versehen ist.

10. Kombinationsgerät nach Anspruch 9, dadurch gekennzeichnet, daß im Träger (14) Rohre (27, 28) zur Aufnahme von Stromversorgungskabeln für Ophthalmometer- und Spaltleuchte (13) vorgesehen sind.

## Claims

1. An instrument for examining eyes comprising in combination a binocular telescopic magnifier with a main objective lens (1) and an ophthalmological examining instrument (6), whereby instead of the main objective lens (1) the ophthalmological examining instrument is insertable, characterized in that the main objective lens (1) of the binocular telescopic magnifier is positioned within a mount (3, 3 ) adapted to rotate about a horizontal axis which runs perpendicular to the optical ray path, said mount (3, 3a) being provided with a connecting means (5) which is adapted to secure simultaneously the ophthalmological instrument (6) and that alternatively the main objective lens (1) or the ophthalmological instrument (6) is insertable in the optical ray path.

2. An instrument according to claim 1, wherein the binocular telescope magnifier cooperates with a slit-lamp (13) when the optical axis of the main objective lens (1) is aligned with the optical ray path.

3. An instrument according to claim 2, wherein the slit-lamp (13) is rotatable about a vertical axis (7).

4. An instrument according to claim 1, wherein said connecting means (5) comprises a dovetail joint.

5. An instrument according to claim 1, wherein to the connecting means (5) an ophthalmometer (6) is attached.

6. An instrument according to claim 1, wherein to the connecting means (5) an edothelium microscope is attached.

7. An instrument according to claim 1, wherein to the connecting means (5) a Placido light source is attached.

8. An instrument according to claim 1, wherein said mount (3, 3a) of said main objective lens (1) is of rectangular circumference and is provided on its upper side with a two-piece protective cover (10, 11), the two-pieces being pivotable each about a horizontal axis (8, 9) such that the front piece (11) covers the main objective lens (1) when this is swung out of the optical ray path.

9. An instrument according to claim 1, wherein the binocular telescope magnifier is attached to a support member (14), said support member (14) being provided with an aperture (15) for receiving a corresponding shaped portion (16) of the ophthalmometric instrument (6).

10. An instrument according to claim 9, wherein said support means (14) is provided with internal passages (27, 28) adapted to receive current supply cables for the illumination of said ophthalmometer and slitlamp (13).

**Revendications**

1. Appareil combiné pour l'examen des yeux, se composant d'une lunette-loupe binoculaire avec objectif principal (1) et d'un appareil d'examen ophtalmologique (6), cet appareil ophtalmologique (6) pouvant être monté à la place de l'objectif principal (1), caractérisé en ce que l'objectif principal (1) de la lunette-loupe binoculaire est installé dans une monture (3, 3a) montée de façon à pouvoir pivoter autour d'un axe horizontal (2) orienté perpendiculairement au trajet des rayons, en ce que la monture (3, 3a) est pourvue d'une pièce de liaison (5) servant à recevoir simultanément l'appareil ophtalmologique (6) et en ce que sélectivement l'objectif principal (1) ou l'appareil ophtalmologique (6) peut être amené par pivotement dans le trajet des rayons.

2. Appareil combiné selon la revendication 1, caractérisé en ce que, lorsque l'objectif principal (1) (fig. 1) a été amené par pivotement dans le trajet des rayons, la lunette-loupe binoculaire coopére avec une lampe à fente (13).

3. Appareil combiné selon la revendication 2, caractérisé en ce que la lampe à fente (13) peut pivoter autour d'un axe vertical (7).

4. Appareil combiné selon la revendication 1, caractérisé en ce que la pièce de liaison (5) est agencée sous forme de pièce de liaison à queue d'aronde.

5. Appareil combiné selon la revendication 1, caractérisé en ce qu'une partie d'ophtalmomètre (6) est fixée sur la pièce de liaison (5).

6. Appareil combiné selon la revendication 1, caractérisé en ce qu'un microscope »Endothel« est fixé sur la pièce de liaison (5).

7. Appareil combiné selon la revendication 1, caractérisé en ce qu'un keratoscope »Placido« est fixé sur la pièce de liaison (5).

8. Appareil combiné selon la revendication 1, caractérisé en ce que la monture (3, 3a) de l'objectif principal (1) a une périphérie rectangulaire et porte sur le côté supérieur (3a) un capot protecteur (10,11) en deux parties, dont les parties peuvent respectivement tourner autour d'un axe horizontal (8, 9), la partie avant (11) recouvrant l'objectif prinzipal (1) lorsque celui-ci est écarté par pivotement du trajet des rayons (fig. 2).

9. Appareil combiné selon la revendication 1, caractérisé en ce que la lunette-loupe binoculaire est fixée sur un support (14), qui est pourvu d'un trou (15) destiné à recevoir une pièce de montage (16), de profil correspondant, de l'appareil ophtalmologique (6).

10. Appareil combiné selon la revendication 9, caractérisé en ce qu'il est prévu dans le support (14) des tubes (27, 28) destinés à recevoir des câbles d'alimentation en courant de la lampe d'ophtalmomètre et de la lampe à fente (13).

# Fig.1

# Fig. 2

# Fig. 3

7